(19)

Europäisches
Patentamt

European
Patent Office

Office européen
des brevets

(11) **EP 4 631 537 A1**

(12) **EUROPEAN PATENT APPLICATION**

(43) Date of publication:
**15.10.2025   Bulletin 2025/42**

(21) Application number: 24169066.8

(22) Date of filing: **08.04.2024**

(51) International Patent Classification (IPC):
**A61L 27/22** (2006.01)      **A61K 31/00** (2006.01)
**A61L 27/54** (2006.01)      **C12N 5/00** (2006.01)

(52) Cooperative Patent Classification (CPC):
**A61L 27/227; A61L 27/54; C12N 5/00**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Leica Microsystems CMS GmbH
35578 Wetzlar (DE)**

(72) Inventor: **Alsheimer, Soeren
35578 Wetzlar (DE)**

(74) Representative: **Paustian & Partner
Patentanwälte mbB
Oberanger 32
80331 München (DE)**

Remarks:
The complete document including Reference
Table(s) and the Sequence Listing(s) can be
downloaded from the EPO website

(54) **THREE-DIMENSIONAL, SELF-ASSEMBLING SCAFFOLD**

(57)    The present disclosure relates to a three-dimensional scaffold comprising, a plurality of scaffold elements, each scaffold element comprising at least one peptide component consisting of a stretch of amino acids and at least two nucleic acid components, wherein the at least two nucleic acid components of the plurality of scaffold elements are configured to mediate the self-assembly of the scaffold.

Figure 1

**Description**

The present disclosure relates to a three-dimensional scaffold comprising, a plurality of scaffold elements, each scaffold element comprising at least one peptide component consisting of a stretch of amino acids and at least two nucleic acid components, wherein the at least two nucleic acid components of the plurality of scaffold elements are configured to mediate the self-assembly of the scaffold.

Background of the Disclosure

Three-dimensional (3D) self-assembling scaffolds play a crucial role in various fields within life sciences.

Scaffolds provide a structural framework that mimics the extracellular matrix (ECM) found in biological tissues. This structural support is essential for tissue engineering and regenerative medicine applications, allowing cells to adhere, proliferate, and differentiate within a 3D environment.

Many self-assembling scaffolds are made from natural or biocompatible materials such as peptides, proteins, or polysaccharides. This biocompatibility reduces the risk of immune rejection or adverse reactions when implanted or used in contact with biological systems.

Self-assembling scaffolds offer tunable properties, including mechanical strength, porosity, degradation rate, and bioactivity. These properties can be precisely engineered to match the requirements of specific tissues or applications, providing versatility and customization.

Scaffolds can encapsulate bioactive molecules such as drugs, growth factors, or genetic material and release them in a controlled manner over time. This controlled release profile enhances the efficacy and safety of therapeutics by maintaining optimal concentrations at the target site while minimizing systemic side effects.

Many self-assembling scaffolds can be fabricated using simple and scalable techniques, including self-assembly, molecular self-organization, or templating methods. This ease of fabrication facilitates mass production and reduces manufacturing costs, making them more accessible for commercial and clinical use.

Self-assembling scaffolds provide a 3D microenvironment that closely resembles the native tissue architecture. This 3D structure allows cells to interact in a more physiologically relevant manner, promoting cell-cell communication, tissue organization, and functional tissue regeneration.

Self-assembling scaffolds can be tailored for a wide range of applications, including tissue engineering, drug delivery, biosensing, diagnostics, and nanotechnology. Their versatility makes them suitable for addressing various challenges in biomedicine and advancing research in diverse fields.

Overall, the advantages of self-assembling scaffolds contribute to their widespread use and importance in advancing biomedical research, enabling innovations in tissue engineering, drug delivery, and regenerative medicine.

There are several different scaffolds reported in the prior art.

Peptides can self-assemble into various nanostructures and scaffolds due to their inherent amphiphilic nature. Peptide-based scaffolds have been utilized in tissue engineering, drug delivery, and regenerative medicine. Examples include peptide amphiphiles (Pas), which can form nanofibers, nanotubes, and other structures.

DNA origami is a technique where single-stranded DNA molecules are folded into precise 3D shapes through base pairing interactions (i.e. hybridization). In addition to DNA origami, DNA brick-based nanostructures have been developed, which self-assemble from smaller DNA bricks and therefore do not require a long scaffold strand. Despite these advances it remains challenging to generate larger DNA nanostructures efficiently. For example, a large DNA-brick based cube with 100nm edge length could be synthesized albeit with an overall efficiency of only -1%. This has prompted the development of Meta-DNA, which is built from smaller DNA nanostructure units that are assembled via pairings of staple strands. These DNA nanostructures can serve as scaffolds for various applications such as drug delivery, biosensing, and nanoelectronics.

Proteins can self-assemble into intricate 3D structures, making them useful scaffolds in biotechnology and biomedicine. Examples include virus-like particles (VLPs), which are self-assembled protein cages derived from viral capsid proteins. VLPs have been investigated for drug delivery, vaccine development, and nanotechnology applications.

Hydrogels are 3D networks of hydrophilic polymers that can absorb and retain large amounts of water. They can self-assemble through various mechanisms such as physical crosslinking, chemical crosslinking, or self-assembly of amphiphilic molecules. Hydrogels have been extensively used in tissue engineering, wound healing, and drug delivery due to their biocompatibility and tunable properties.

Nanocellulose, derived from plant sources, can self-assemble into nanofibrils and nanocrystals.

These nanocellulose scaffolds have gained attention in tissue engineering, wound dressing, and as drug delivery carriers due to their biocompatibility, mechanical strength, and tunable properties.

Peptide amphiphile molecules can self-assemble into nanofibers with precise control over their structure and function. These nanofiber scaffolds have been investigated for applications in tissue regeneration, neural tissue engineering, and drug delivery.

Various synthetic polymers can self-assemble into 3D structures through non-covalent interactions such as hydrogen bonding, π-π stacking, and hydrophobic interactions. These self-assembling polymer scaffolds have been explored in drug delivery, tissue engineering, and nanotechnology applications.

These examples highlight the diverse range of self-assembling scaffolds used in life sciences and related fields, each with unique properties and applications.

While self-assembling scaffolds offer many advantages, they also come with certain limitations and disadvantages:

Controlling the degradation rate of scaffolds to match tissue regeneration can be challenging. Scaffolds may degrade too quickly, leading to inadequate support for tissue growth, or too slowly, causing prolonged inflammation or foreign body reactions.

Some self-assembling scaffolds may lack sufficient mechanical strength or stiffness to withstand physiological loads, particularly in load-bearing tissues. Achieving the desired mechanical properties while maintaining biocompatibility and biodegradability can be difficult.

Although many self-assembling scaffolds are made from biocompatible materials, they may still elicit immune responses in some individuals, leading to inflammation, fibrosis, or rejection. Immune reactions can compromise the functionality and integration of the scaffold within the host tissue.

Fabricating self-assembling scaffolds with precise control over their structure and properties can be complex and require sophisticated techniques. Ensuring reproducibility and scalability of fabrication processes may pose challenges, particularly for clinical translation and commercialization.

While some scaffolds can incorporate bioactive molecules for enhanced functionality, achieving sustained and controlled release of these molecules can be difficult. Maintaining bioactivity over time and ensuring appropriate spatial distribution within the scaffold may also be challenging. Introducing novel biomaterials and scaffolds into clinical practice requires rigorous regulatory approval processes to ensure safety and efficacy. Meeting regulatory standards for biocompatibility, sterility, and clinical performance adds time and cost to the development and commercialization of new scaffolds.

Ensuring proper integration of the scaffold with host tissue and promoting functional regeneration remains a significant challenge. Scaffold design must consider factors such as cell adhesion, migration, vascularization, and innervation to support tissue remodeling and restore tissue function effectively.

Developing and manufacturing self-assembling scaffolds using advanced biomaterials and fabrication techniques can be expensive. High production costs may limit their widespread adoption, particularly in resource-limited settings or for large-scale applications.

Thus, a need exists in the prior art to overcome said disadvantages. The three-dimensional, self-assembling scaffolds according to embodiments of the present disclosure do overcome these disadvantages thereby preserving the advantages associated with such scaffolds.

Summary

In a first aspect the present disclosure relates to a three-dimensional scaffold comprising, a plurality of scaffold elements, each scaffold element comprising at least one peptide component consisting of a stretch of amino acids and at least two nucleic acid components, wherein the at least two nucleic acid components of the plurality of scaffold elements is configured to mediate the self-assembly of the scaffold.

In a second aspect the present disclosure relates to a method for producing a three-dimensional scaffold comprising the steps of:

    a. Providing a plurality of scaffold elements comprising at least one peptide component and at least two nucleic acid components;

    b. Wherein, optionally, at least one of the scaffold elements further comprises a binding element selected from a nucleic acid based binding region and/or a peptide based binding region;

    c. Bringing the scaffold elements in solution, whereby the self-assembly of the scaffold elements takes place, and

    d. Obtaining the resulting three-dimensional scaffold.

In a third aspect the present disclosure relates to a three-dimensional scaffold produced by the disclosed methods.

In a fourth aspect the present disclosure relates to uses of the disclosed three-dimensional scaffold selected from the group consisting of use in biological imaging and labeling, use in templates for material synthesis, use in molecular sensing, use in diagnostic tools, use in molecular robotics and computing, use in synthetic biology, use in bottom-up nanofabrication, use in nanoscale devices, use in bioprocessing, and use in bioprinting, as well as combinations thereof.

In a fifth aspect the present disclosure relates to the three-dimensional scaffold disclosed hereinunder, for use as a medicament, such as in tissue and/or cellular repair, tissue and/or cellular engineering, drug delivery, wound treatment, bone reconstruction, building, also partially, artificial organs, as well as combinations thereof.

In a sixth aspect the present disclosure relates to a kit comprising the three-dimensional scaffold as disclosed hereinunder, further comprising an item selected from a buffer, a package leaflet, an applicator, an administration device, a mixing device, a manual, a device for induction of polymerization, a dye, and a hydrogel-matrix, as well as combinations thereof.

Brief Description of the Figures

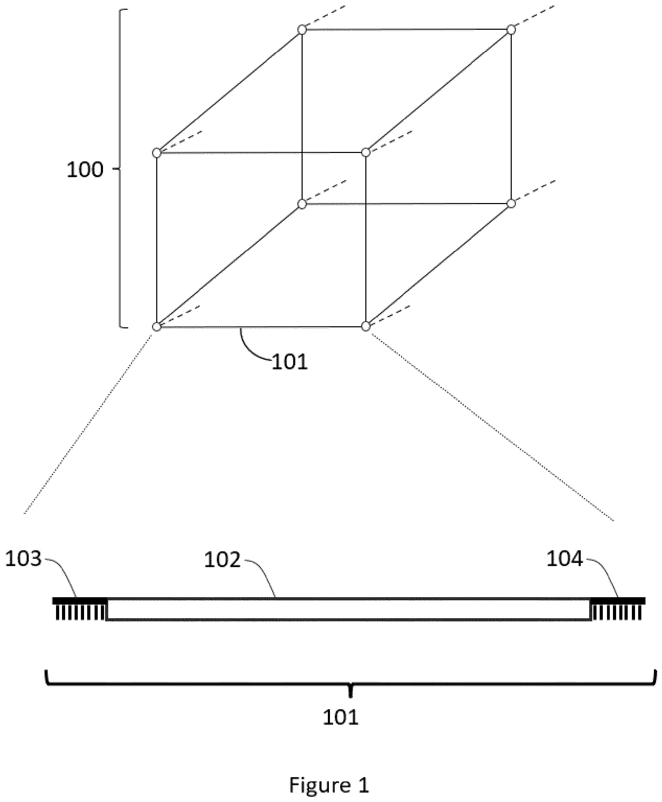

Figure 1 - Schematic depiction of the three-dimensional, self-assembling scaffold according to embodiments of the present disclosure. The three-dimensional scaffold comprises a plurality of scaffold elements (101) which may be identical or different from each other. Each scaffold element comprising at least one peptide component (102) consisting of a stretch of amino acids and at least two nucleic acid components (103,104), wherein the at least two nucleic acid components (103,104) of the plurality of scaffold elements (101) is configured to mediate the self-assembly of the scaffold (100).

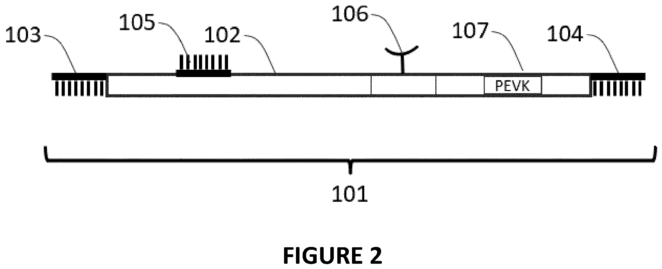

Figure 2 - Schematic depiction of a scaffold element (101). The element may comprise internal nucleic acid -based binding region(s) (105), and/or internal peptide-based binding region(s) (106) which facilitate further binding to a target selected from the group consisting of nucleic acid, peptide, protein, inorganic molecule (e.g. toxin or radionuclide), organic molecule (e.g. microplastic, dye or fluorescent), sugar and/or lipid. The scaffold element (101) may also comprise one or more PEVK-motifs in its peptide component (102).

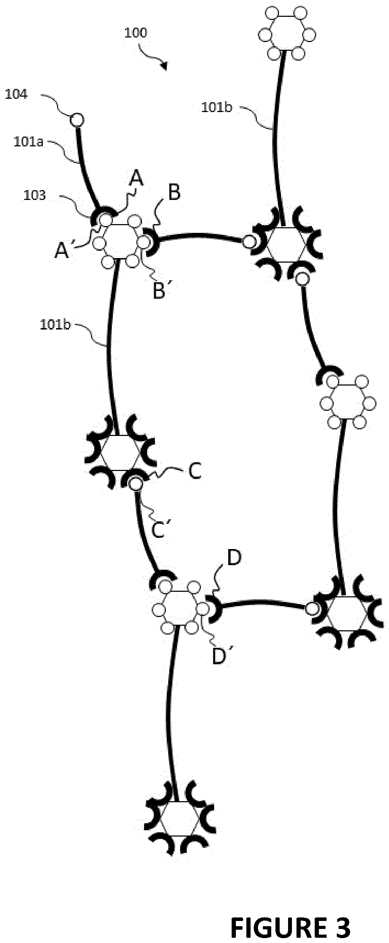

Figure 3 - Another schematic depiction of the three-dimensional, self-assembling scaffold according embodiments of the present disclosure. Depicted is the interaction of the different elements. The at least two nucleic acid components (103,104) - in one embodiment being located at the ends of the scaffold elements (101a) comprise nucleic acid regions (e.g. A, B) which specifically hybridize with nucleic acid regions (e.g. A', B') on other scaffold elements (101b), thereby facilitating a directed self-assembly. The at least two nucleic acid components (103,104) may be present in several copies at the ends of the scaffold elements (101a, 101b).

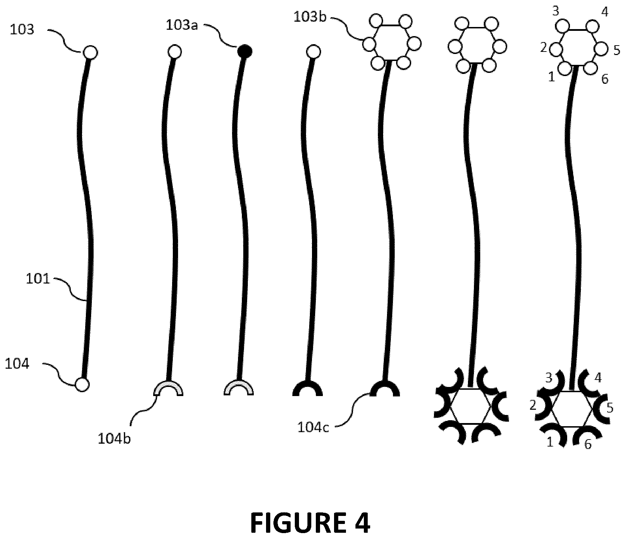

Figure 4 - Schematic depiction of different scaffold elements (101). Depending on the envisaged three-dimensional structure of the scaffold, the at least two nucleic acid components (103,103a, 103b, ..., 104, 104a, 104b, ...) of the scaffold elements, in one embodiment located at the ends thereof, may be designed for different functional purposes. Hybridization strength and specificity may be facilitated by the composition ("barcode") and/or length of the complementary nucleic acid sequences and/or by copy numbers (1, 2, 3, ...) of the nucleic acid component(s).

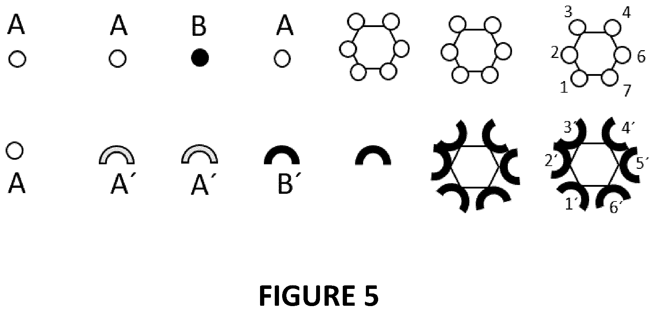

Figure 5 - Schematic representations of complementary binding elements. Nucleic acid sequence ("barcode") "A" may hybridize to barcode A' for example. Alternatively, A may be an affinity partner A binding to an affinity partner A'.

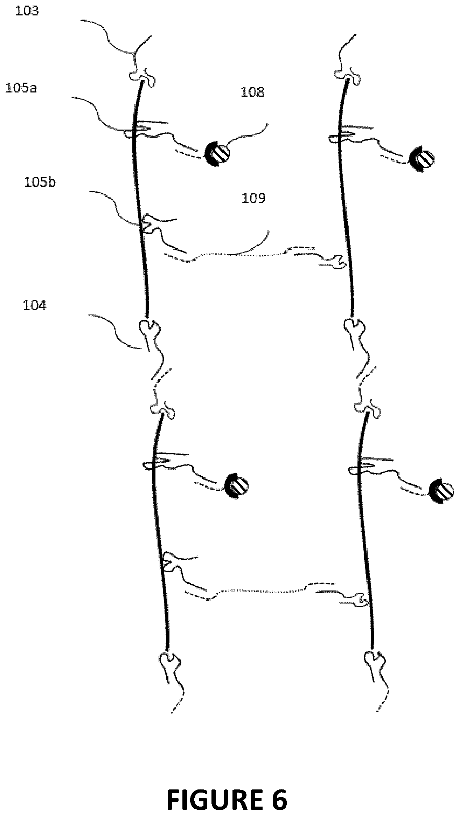

Figure 6 - Another schematic depiction of the three-dimensional, self-assembling scaffold according to embodiments of the present disclosure. The different scaffold elements may additionally comprise internal nucleic acid-based binding region(s) (105), and/or internal peptide-based binding region(s) (106) which facilitate further binding to a target selected from the group consisting of nucleic acid, peptide, protein, inorganic molecule (e.g. toxin or radionuclide), organic molecule (e.g. microplastic, dye or fluorescent), sugar and/or lipid. Depicted is an example of an aptamer (108) comprising a region which hybridizes with the internal nucleic acid-based binding region (105a) and further binds to a protein target. Depicted is also an example of cross-linking nucleic acid component (109) which comprises a region which hybridizes with the internal nucleic acid-based binding regions (105b) of two different scaffold elements.

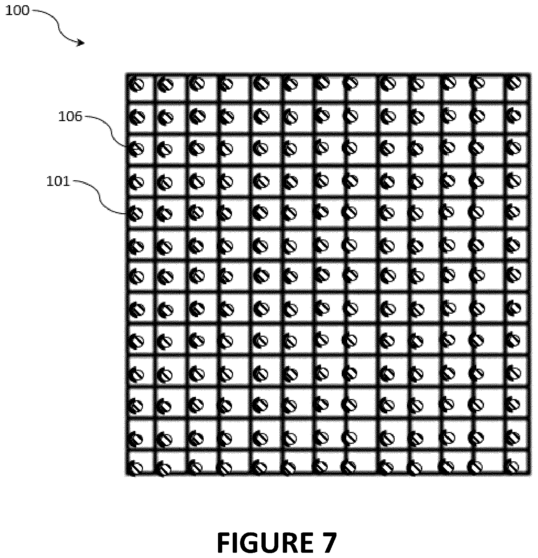

Figure 7 - Another schematic depiction of the three-dimensional, self-assembling scaffold according to embodiments of the present disclosure. Depicted is the regular three-dimensional structure and an example where the different scaffold elements (101) comprise internal peptide-based binding region (106) which facilitate further binding to a target selected from the group consisting of nucleic acid, peptide, protein, inorganic molecule (e.g. toxin or radionuclide), organic molecule (e.g. microplastic, dye or fluorescent), sugar and/or lipid.

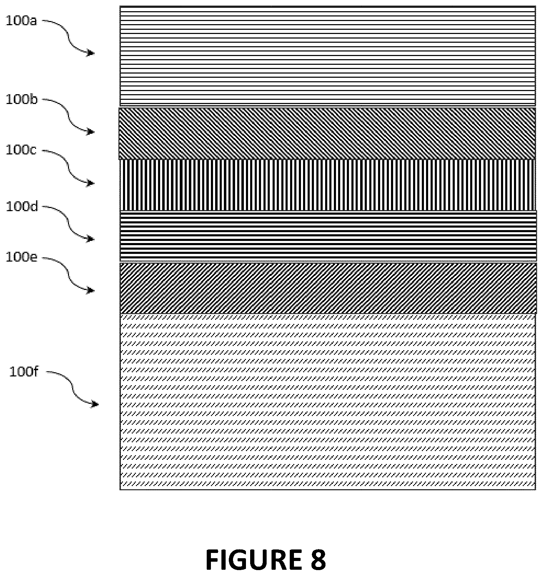

Figure 8 - Schematic depiction of several layers of the three-dimensional, self-assembling scaffold according to embodiments of the present disclosure (100a - 100f). Especially in case of tissue engineering and/or sophisticated filter-systems the combination of several layers of the three-dimensional, self-assembling scaffold according to the disclosure may become useful. This may be facilitated by combining three-dimensional, self-assembling scaffolds with certain physical (i.e. mesh structure, pore size, PDI) and/or different biochemical properties with each other by also incorporating specific binding sections for other three-dimensional, self-assembling scaffolds at their interfaces.

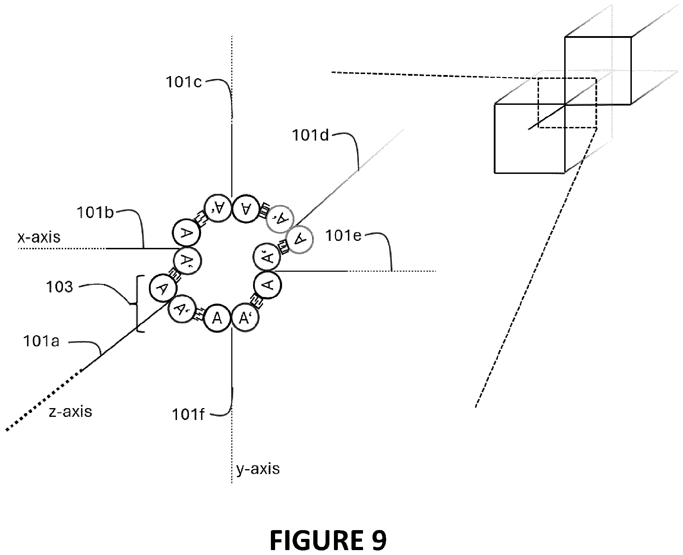

Figure 9 - Schematic depiction of the "corners" of the three-dimensional, self-assembling scaffold according to embodiments of the present disclosure. The at least two nucleic acid components (103,104) at the ends of the scaffold elements (101a-f) comprise at least two different nucleic acid regions (e.g. A, A') which specifically hybridize with nucleic acid regions (e.g. A', A) on other scaffold elements (101a-f), thereby facilitating a directed self-assembly.

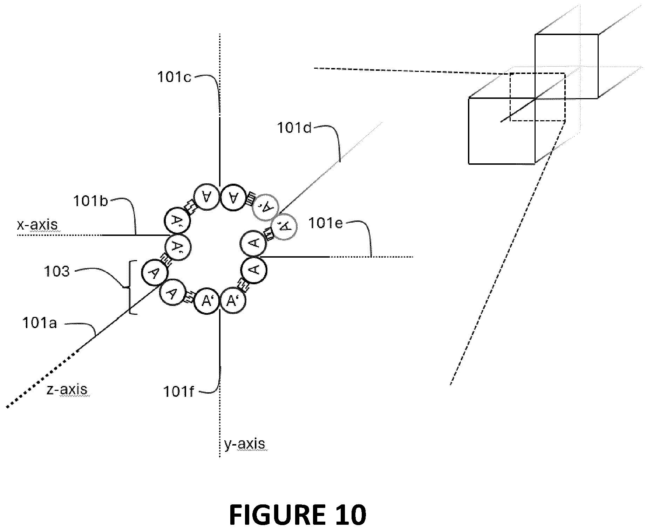

Figure 10 - Schematic depiction of the "corners" of the three-dimensional, self-assembling scaffold according to embodiments of the present disclosure. The figure is a variation of figure 9. The at least two nucleic acid components (103,104) at the ends of the scaffold elements (101a-f) comprise at least two of the same nucleic acid regions (e.g. A, A or A',A') which specifically hybridize with nucleic acid regions on other scaffold elements (101a-f), thereby facilitating a directed self-assembly.

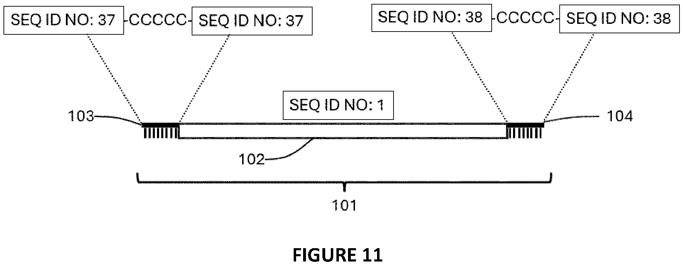

Figure 11 - Schematic depiction of a typical scaffold element (101) according to the present disclosure. The scaffold element (101) comprises nucleic acid regions at each end which are two copies of SEQ ID NO: 37 or SEQ ID NO: 38, in one embodiment interspersed by a nonsense nucleic acid region of 5 nucleotides (i.e. CCCCC).

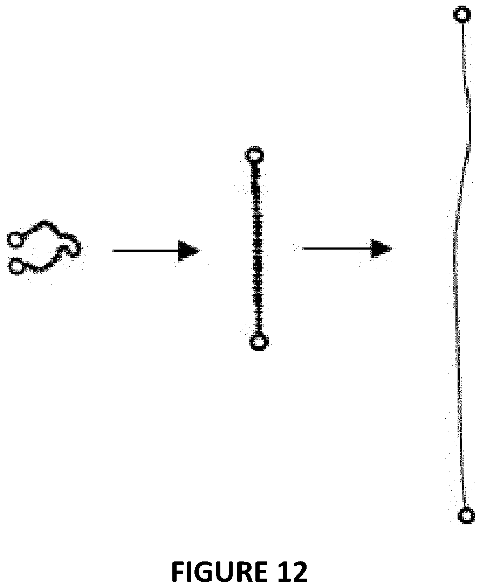

Figure 12 - Schematic depiction of the "expandability" of a scaffold element. In one embodiment the scaffold element, in one embodiment the peptide component of the scaffold element, can be expanded by a factor of at least 2, at least 5, at least 10, at least 100, or at least 1,000.

Reference-numbers of the figures:

100 - three-dimensional, self-assembling scaffold (elements 100a - 100f are used in some figures to depict different three-dimensional, self-assembling scaffolds)

101 - scaffold element, suitable to form the three-dimensional, self-assembling scaffold (elements 101a, 101b, etc. are used in some figures to depict different scaffold elements)

102 - peptide component of the scaffold element

103 - first nucleic acid component of the scaffold element (elements 103a, 103b, etc. are used in some figures to depict different first nucleic acid components and variants); "1, 2, 3, 4, ..." in figure 4 refers to several copies of the same or different first nucleic acid component.

104 - second nucleic acid component of the scaffold element (elements 104a, 104b, etc. are used in some figures to depict different second nucleic acid components and variants); "1, 2, 3, 4, ..." in figure 4 refers to several copies of the same or different second nucleic acid component.

105 - internal nucleic acid -based binding region which is different form the elements (103, 104) and hybridizes to nucleic acid targets.

106 - internal peptide-based binding region which binds a target selected from the group consisting of nucleic acid, peptide, protein, inorganic molecule (e.g. toxin or radionuclide), organic molecule (e.g. microplastic, dye or fluorescent), sugar and/or lipid.

107 - amino acid motif consisting of proline (P), glutamate (E), valine (V), and lysine (K) (PEVK)

108 - example of a molecule which may hybridize with internal nucleic acid -based binding region (105); depicted is an aptamer which allows binding to a target selected from the group consisting of nucleic acid, peptide, protein, inorganic molecule (e.g. toxin or radionuclide), organic molecule (e.g. microplastic, dye or fluorescent), sugar and/or lipid.

109 - example of a molecule which may hybridize with internal nucleic acid -based binding region (105); depicted is another nucleic acid which forms an additional intra-molecular bridge to another scaffold element.

A, A' - Depiction of essentially complementary nucleic acid sequences able to specifically hybridize with each other (same for B, B'; C, C', etc.)

Detailed Description

The present disclosure pertains to a three-dimensional scaffold comprising, a plurality of scaffold elements, each scaffold element comprising at least one peptide component consisting of a stretch of amino acids and at least two nucleic acid components, wherein the at least two nucleic acid components of the plurality of scaffold elements is configured to mediate the self-assembly of the scaffold.

Such a three-dimensional scaffold may also be called a "hybrid scaffold" since it comprises both peptide as well as nucleic acid elements. As such it combines the advantages of both "worlds", i.e. the versatility, flexibility, and strength of a peptide backbone with the programmability and abilities for self-assembly of nucleic acids.

**Peptide component**

The peptide component serves several purposes in terms of rigidity, flexibility, elasticity, and size of the three-dimensional scaffold, which influence the design and selection of the peptide. Thus, the at least one peptide component comprises at a molecular weight of 2.5 MDa at least one feature selected from the group consisting of:

a. a longitudinal tensile strength of at least 350 MPa, of at least 375 MPa, of at least 400 MPa, of at least 425 MPa, of at least 450 MPa, of at least 475 MPa, of at least 500 MPa, or of at least 550 MPa. In one embodiment the longitudinal tensile strength is 800 MPa or less, 700 MPa or less, 600 MPa or less, 590 MPa or less, 580 MPa or less, or 560 MPa or less. In one embodiment the longitudinal tensile strength is from 350 MPa to 800 MPa, from 400 MPa to 700 MPa, from 500 MPa to 600 MPa, or from 525 MPa to 575 MPa.

b. a tensile modulus of at least 3.5 GPa, of at least 3.75 GPa, of at least 4.0 GPa, of at least 4.25 GPa, of at least 4.5 GPa, of at least 4.75 GPa, of at least 5.0 GPa, or of at least 5.25 GPa. In one embodiment the tensile modulus is 10.0 GPa or less, 9.0 GPa or less, 8.0 GPa or less, 7.0 GPa or less, or 6.0 GPa or less. In one embodiment the tensile modulus is from 3.5 GPa to 10.0 GPa, from 4.0 GPa to 9.0 GPa, from 4.5 GPa to 8.0 GPa, from 5.0 GPa to 7.0 GPa, or from 5.5 GPa to 6.0 GPa.

c. an extensibility of at least 45%, of at least 50%, of at least 55%, of at least 60%, of at least 65%, or of at least 70%. In one embodiment the extensibility is 100% or less, 95% or less, 90% or less, or 85% or less. In one embodiment the extensibility is from 45% - 100%, from 50% - 95%, from 55% - 90%, from 60% - 85%, or from 65% - 80%.

d. a toughness of at least 120 Mj/m$^3$, of at least 130 Mj/m$^3$, of at least 140 Mj/m$^3$, of at least 150 Mj/m$^3$, of at least 160 Mj/m$^3$, of at least 170 Mj/m$^3$, of at least 180 Mj/m$^3$, or of at least 190 Mj/m$^3$. In one embodiment the toughness is 250 Mj/m$^3$ or less, 240 Mj/m$^3$ or less, 230 Mj/m$^3$ or less, 220 Mj/m$^3$ or less, or 210 Mj/m$^3$ or less. In one embodiment the toughness is from 120 Mj/m$^3$ to 250 Mj/m$^3$, from 130 Mj/m$^3$ to 240 Mj/m$^3$, from 140 Mj/m$^3$ to 230 Mj/m$^3$, from 150 Mj/m$^3$ to 220 Mj/m$^3$, or from 160 Mj/m$^3$ to 210 Mj/m$^3$.

e. the peptide component (102) having a length of at least 0.5 μm, of at least 0.6 μm, of at least 0.7 μm, of at least 0.8 μm, of at least 0.9 μm, of at least 1.0 μm, of at least 1.1 μm, of at least 1.2 μm, of at least 1.3 μm, or of at least 1.5 μm. In one embodiment the peptide component (102) has a length of 5 μm or less, 4 μm or less, 3 μm or less, 2 μm or less, or 1.8 μm or less. In one embodiment the peptide component (102) has length from 0.5 μm to 5 μm, from 0.6 μm to 4 μm, from 0.7 μm to 3 μm, from 0.8 μm to 2 μm, or from 1.0 μm to 1.5 μm.

f. Comprising at least 3,000 amino acids, at least 5,000 amino acids, at least 10,000 amino acids, at least 15,000 amino acids, at least 20,000 amino acids, at least 21,000 amino acids, at least 22,000 amino acids, at least 22,500 amino acids, at least 22,700 amino acids, at least 22,720 amino acids, at least 22,800 amino acids, or at least 22,900 amino acids. In one embodiment the peptide component (102) comprises 50,000 amino acids or less, 45,000 amino acids or less, 40,000 amino acids or less, 38,000 amino acids or less, or 37,000 amino acids or less. In one embodiment the peptide component (102) comprises from 3,000 to 50,000 amino acids, from 5,000 to 45,000 amino acids, from 10,000 to 40,000 amino acids, or from 20,000 to 38,000 amino acids.

Obviously, the molecular weight of the peptide component does not have to be 2.5 MDa, this value is only intended to enable the comparison of different structural proteins and is therefore to be understood as the size at which the physical parameters mentioned are determined using the usual test methods. In some embodiments the peptide component may comprise two or more copies and/or partial copies of an amino acid sequence in order to achieve the desired length. In some embodiments the peptide component may comprise two or more copies and/or partial copies of a peptide selected from SEQ ID NO: 1 - 36, as well as variants and isoforms thereof.

There are naturally occurring structural proteins that, due to their role in nature, already have the necessary properties that are also desirable in the disclosed three-dimensional scaffold.

Such structural proteins may for example be cytoskeletal proteins that play crucial roles in maintaining the structural integrity of cells, particularly in the context of cell shape, stability, and membrane organization, proteins of the extracellular matrix, proteins of the microfilaments-family, and/or part of muscle fibers. One group of such naturally occurring structural proteins consists of nebulin, obscurin, dystrophin, and titin, as well as combinations thereof.

Thus, in one embodiment the at least one peptide component (102) is selected from nebulin, obscurin, dystrophin, and titin, as well as combinations thereof.

In one embodiment a protein of SEQ ID NO: 1 - 36 is preferred. In one embodiment the peptide component comprises a sequence with a sequence identity to the sequences selected from the group comprising SEQ ID NO: 1 to 36 of at least 80%, at least 85%, at least 90%, at least 95%, at least 98%, at least 99%, at least 99.9%, as well as variants, fragments and isoforms thereof. In one embodiment the protein is a titin (SEQ ID NO: 1 - 13), or a sequence with a sequence identity to the sequences selected from the group comprising SEQ ID NO: 1 to 13 of at least 80%, at least 85%, at least 90%, at least 95%, at least 98%, at least 99%, at least 99.9%, as well as variants, fragments and isoforms thereof.

It is mandatory for the correct function of the scaffold that no peptide-nucleic acid variants (PNA) are comprised in the peptide component (102). Thus, in one embodiment the peptide component (102) consists only of a stretch of amino acids connected by peptide-bonds. Thus, in one embodiment the peptide component (102) is free of any PNA.

In one embodiment the peptide component comprises at least 100, at least 150, at least 200, at least 250, at least 300, at least 350, or at least 400 Ig-like domains.

In one embodiment the peptide component comprises at least 50, at least 75, at least 80, at least 85, at least 90, at least 95, at least 100, at least 105, at least 110, at least 115, at least 125, at least 130, at least 135 type I fibronectin type III domains.

In one embodiment the peptide component comprises at least 50, at least 75, at least 80, at least 85, at least 90, at least 95, at least 100, at least 105, at least 110, at least 115, type II immunoglobulin domains.

In one embodiment the peptide component comprises at least 10, least 12, least 14, least 16, least 18, least 20, least 22, least 24 proline-glutamate-valine-lysine (PEVK)-motifs.

In one embodiment the peptide component (102) may further comprise at least one peptide-based binding region (106). The at least one peptide-based binding region (106) may be selected from the group consisting of an antigen binding peptide such as an antibody, Fab-fragment and/or svFc-fragment, a peptide aptamer, a DARPin (designed ankyrin repeat protein), an affimer, a nanobody, a click-chemistry-based ligand, a polymeric binder, an affinity tag [HA, Myc, FLAG], protein A, protein G, streptavidin and an aptazyme, a helix-turn-helix (HTH) domain, a zinc-finger domain, a leucine-zipper domain, a basic helix-loop-helix (bHLH) domain, homeodomain, AT-hook motif, arginine-glycine-rich (RGG) domain, a RNA-recognition motif (RRM), cys-2-his-2-zinc finger ($C_2H_2$), and a polycomb repressive complex (PRC1) ring finger domain, as well as combinations thereof.

**Nucleic acid component**

The three-dimensional scaffold of the present disclosure comprises a plurality of scaffold elements, each scaffold element may comprise at least two nucleic acid components.

The at least two nucleic acid components comprise a first nucleic acid component and second nucleic acid component, the second nucleic acid component being different to the first nucleic acid component, wherein the first and second nucleic acid component are configured to hybridize to different targets.

In one embodiment the nucleic acid components of each scaffold element are configured to not bind to itself, i.e. nucleic acid component of a first scaffold element (e.g. 101a) will not bind to nucleic acid component of a second scaffold element (e.g. 101b). However, in one embodiment, nucleic acid component of a first scaffold element (e.g. 101a) may bind to nucleic acid component of a second scaffold element (e.g. 101b).

In one embodiment the nucleic acid components are positioned at each end of the scaffold element, wherein "end" may be in one embodiment the most distal part of each scaffold element, but in some embodiments includes also configurations where the nucleic acid components are within 10, 20, 30, 40 or 50 amino acids and/or 10, 20, 30, 40 or 50 nucleic acids of one of the ends of the scaffold element.

Further, within each of the at least two nucleic acid components different hybridization regions can be defined, which allow the specific hybridization with different targets. For example, a nucleic acid component may comprise a nucleic acid sequence A which enables the hybridization with nucleic acid sequence A', but may additionally comprise a nucleic acid sequence B which enables the hybridization with nucleic acid sequence B', etc.

In one embodiment the at least two nucleic acid components may be encoded to specifically hybridize with at least one of the nucleic acid components of another scaffold element, thereby mediating the self-assembly of the scaffold.

In general, the nucleic acid components are designed to facilitate a three-dimensional structure. Each "corner" of the three-dimensional scaffold needs to provide at least two specific contacts to the at least five other different scaffold elements (cf. figure 9). This can be achieved by a minimum of at least two different nucleic acid sequences (A, A') per nucleic acid components (i.e. per "end" of each scaffold element), wherein A hybridizes with A'. However, in order to facilitate the self-assembly in a more controlled fashion and/or more complex structures, the different nucleic acid components may comprise more than two sequences and/or more than two copy numbers of the same or different sequences.

In one embodiment the nucleic acid sequence to be used for A, A' B, B', etc. should be unique sequences which are not to be found in the target tissue or subject. This may be achieved by synthetic sequences and/or by using naturally occurring unique sequences, such as for example a part of the nucleic acid sequence of human hemoglobin subunit beta (cf. SEQ ID NO: 37).

Thus, in one embodiment each of the at least two nucleic acid components may comprise two different sequences A and A', or one sequence pair, which hybridize to each other. In further embodiments each of the at least two nucleic acid components may comprise at least 2, at least 3, at least 4, at least 5, at least 6, at least 7, at least 8, at least 9, at least 10, at least 11, or at least 12 different sequence pairs which hybridize to each other.

In order to facilitate specific hybridization, each of the sequences (i.e. A, A', B, B', etc.) comprises at least 9 nucleotides, at least 10 nucleotides, at least 11 nucleotides, at least 12 nucleotides, at least 13 nucleotides, at least 14 nucleotides, at least 15 nucleotides, at least 16 nucleotides, at least 17 nucleotides, at least 18 nucleotides, at least 19 nucleotides, or at least 20 nucleotides. Less nucleotides result in unspecific hybridization, more nucleotides do normally not improve the hybridization, but are more difficult to produce. However, in exceptional cases longer sequences may be incorporated for specific applications.

In another embodiment the at least two nucleic acid components are covalently conjugated to the peptide component of the scaffold element. In another embodiment the at least two nucleic acid components are attached to the peptide component of the scaffold element indirectly via an affinity interaction (e.g. aptamer-based, nanobody-based, Streptavidin-biotin).

In another embodiment the scaffold element may comprise at least one further nucleic acid based binding region. In contrast to the at least two nucleic acid components the at least one further nucleic acid based binding region is positioned more in the middle of the scaffold element. In one embodiment it may be encompassed on both sides by the peptide component of the scaffold element, basically diving the peptide component in two parts.

In one embodiment the at least two nucleic acid components and/or at least one nucleic acid based binding region are selected from the group consisting of desoxyribonucleic acid strands (DNA), L-DNA, D-DNA, PT-DNA, ribonucleic acid strands (RNA), xeno nucleic acid strands (XNA), peptide nucleic acid strands (PNA), locked nucleic acid strands (LNA), and morpholino nucleic acid strands (MNA), as well as combinations thereof.

In another embodiment the at least two nucleic acid component and/or at least one nucleic acid based binding region may comprise at least one structural element selected from a nucleic acid origami-element, such as a DNA tile, a DNA brick, a RNA tile, a RNA brick, a XNA tile, a XNA brick, a PNA tile, a PNA brick, a LNA tile, a LNA brick, a MNA tile, and a MNA brick, as well as combinations thereof, and/or a binding element selected from an aptamer, a DNAzyme (Deoxy-ribozyme), a riboswitch, and an aptazyme, as well as combinations thereof.

In one embodiment of the present disclosure the nucleic-acid elements are "programmable".

That is, they are designed to carry a certain "barcode", i.e. a specific nucleic acid sequence which allows the control and regulation of the self-assembly of the scaffold. They also allow the fine-tuning of the features of the scaffold, such as the mesh-size, polydispersity index (PDI), overall strength of the scaffold, water-binding abilities, binding abilities to target molecules, crosslinking, etc.

In one embodiment the present disclosure also encompasses expression vectors for the production of the scaffold element(s) in bacteria and/or yeast. Such a vector usually comprises:

- A promoter sequence, usually derived from a strong and constitutively active promoter such as the CMV (cytomegalovirus) promoter or the SV40 (simian virus 40) promoter, drives transcription of the gene of interest. The promoter initiates the transcription process by recruiting RNA polymerase and other transcription factors to the gene's transcription start site.

- The expression vector contains the gene encoding for the scaffold element(s) (101) (or at least parts thereof). The gene is typically inserted into the vector using specific restriction enzyme sites or other cloning techniques.

- A selectable marker gene, such as an antibiotic resistance gene (e.g., ampicillin resistance gene, kanamycin resistance gene) or a reporter gene (e.g., GFP, $\beta$-galactosidase), is included in the vector to facilitate the selection and identification of host cells that have successfully taken up the vector. Cells that have incorporated the vector can be selected by culturing them in a medium containing the corresponding selection agent.

- The origin of replication is a DNA sequence that allows the vector to replicate independently of the host organism's chromosomal DNA. It ensures that the vector is replicated and maintained in host cells during cell division.

- A polyadenylation signal sequence, typically derived from the bovine growth hormone (BGH) or SV40, is included downstream of the gene of interest. This sequence directs the addition of a polyadenylate (poly-A) tail to the mRNA transcript, which stabilizes the mRNA and enhances its translation efficiency.

- A multiple cloning site, also known as a polylinker or restriction site, is a region within the vector that contains multiple unique restriction enzyme recognition sites. These sites allow for the insertion of the gene of interest into the vector at specific locations, facilitating cloning and manipulation of the vector.

- Optionally, expression vectors may include additional enhancer elements or regulatory sequences to enhance gene expression levels or tissue-specific expression patterns.

In one embodiment the disclosure encompasses also a microorganism, i.e. bacterium and/or yeast, comprising such a vector, either stably and/or transiently incorporated.

**Three-dimensional scaffold**

In one embodiment the three-dimensional scaffold may be further characterized by at least one of the features:

a. Having a peptide content of at least 1 wt.-%, at least 2 wt.-%, at least 5 wt.-%, at least 10 wt.-%, at least 20 wt.-%, or at least 30 wt.-%. In one embodiment having a peptide content of 90 wt-% or less, of 85 wt-% or less, of 80 wt-% or less, of 75 wt-% or less, of 70 wt-% or less, of 65 wt-% or less, or of 60 wt-% or less. In one embodiment having a peptide content from 1% to 40% (w/v), from 5% to 35% (w/v), from 8% to 30% (w/v), from 10% to 25% (w/v), or from 15% to 20% (w/v); and/or

b. Having a nucleic acid content of at least 0,01 wt.-%, at least 0,02 wt.-%, at least 0,05 wt.-%, at least 0,1 wt.-%, at least 0,5 wt.-%, or at least 1 wt.-%. In one embodiment having a nucleic acid content of 40 wt-% or less, of 35 wt-% or less, of 30 wt-% or less, of 28 wt-% or less, of 25 wt-% or less, of 22 wt-% or less, or of 20 wt-% or less. In one embodiment having a nucleic acid content from 0,01% to 30% (w/v), from 0,05% to 20% (w/v), from 0,1% to 15% (w/v), from 0,5% to 10% (w/v), or from 1 % to 5% (w/v).

b. Having a water content of at least 10 vol.-%, at least 15 vol.-%, at least 20 vol.-%, at least 25 vol.-%, at least 30 vol.-%, at least 35 vol.-%, or at least 40 vol.-%. In one embodiment having a water content of 99 vol.-% or less, of 98 vol.-% or less, of 95 vol.-% or less, of 90 vol.-% or less, of 85 vol.-% or less, of 80 vol.-% or less, of 75 vol.-% or less, of 70 vol.-% or less, or of 65 vol.-% or less. In one embodiment having a water content from 10 to 99 vol.-%, or from 20 to 98 vol.-%, from 30 to 95 vol.-%, from 40 to 90 vol.-%, from 50 to 85 vol.-%, from 60 to 80 vol.-%,

c. having a programmable 3-d pattern, wherein the nucleic acid components; and/or the peptide component are designed to form a specific 3-d pattern based on nucleic acid - nucleic acid, protein - protein and/or nucleic acid - protein interactions, wherein the features of the 3-d pattern are selected from:

- Porous Networks: Porosity is a key feature for nutrient and waste exchange, mimicking the porous structure of natural ECM. The size, shape, and distribution of the pores can be tailored to match the requirements of different cell types and tissues. Porous scaffolds can be created using techniques such as gas foaming, salt leaching, freeze-drying, and electrospinning.

  - Fiber Networks: Electrospinning is a popular technique for creating scaffolds that consist of intertwined fibers, mimicking the fibrous component of the ECM. The fibers can vary in diameter from nanometers to micrometers, and the density and orientation of the fibers can be controlled to influence cell behavior.

  - Layered Structures: Using techniques like 3D printing (bioprinting) or layer-by-layer assembly, scaffolds can be fabricated with precise layered structures that can mimic the stratified organization of tissues such as skin or cartilage.

  - Honeycomb Patterns: Honeycomb-like patterns, characterized by regular hexagonal pores, are favorable for certain types of tissue engineering due to their high porosity and uniform pore distribution, which can enhance cell seeding and nutrient diffusion.

  - Spherical and Ellipsoidal Structures: Spheroid or ellipsoid structures within scaffolds can be particularly useful for the cultivation of stem cells or the formation of tissue spheroids, which are important in organoid development and cancer research.

  - Vascular-like Networks: Creating scaffolds with embedded vascular-like channels is crucial for engineering tissues with high metabolic demands, such as liver or heart tissue. These patterns ensure the transport of nutrients and oxygen throughout the scaffold, facilitating the survival of embedded cells.

- Gradient Structures: Some tissues require gradients of biochemical cues or physical properties (e.g., stiffness) across the scaffold to guide tissue formation. Advanced fabrication techniques can create scaffolds with spatial gradients to mimic these natural tissue characteristics.

d. being a programmable self-assembling hydrogel, in one embodiment having a water content of at least 30% (w/v), at least 40% (w/v), at least 50% (w/v), at least 60% (w/v), at least 70% (w/v), at least 80% (w/v), at least 90% (w/v), at least 95% (w/v), or at least 99% (w/v),

e. which fills a volume of at least 0.001 $\mu m^3$, at least 1$\mu m^3$, at least 1000$\mu m^3$, at least 0.001mm$^3$, at least 1mm$^3$, at least 1000mm$^3$, at least 1cm$^3$, at least 1000cm$^3$, or at least 0.0001m$^3$,

f. which consists of a number of at least 10, at least 100, at least 1000, at least 10,000, or at least 100,000 scaffold elements,

g. which is embedded in a hydrogel or polymer matrix that can be expanded by a factor of at least 2, at least 5, at least 10, at least 100, or at least 1000;

h. being biodegradable within less than 1 year, less than 6 months, less than 5 months, less than 4 months, less than 3 months, less than 2 months, or less than 1 month.

In one embodiment the disclosure pertains to a method for producing a three-dimensional scaffold comprising the steps of

a. Providing a plurality of scaffold elements (101) comprising at least one peptide component (102) and at least two nucleic acid components (103,104);

b. Wherein, optionally, at least one of the scaffold elements (101) further comprises a binding element selected from a nucleic acid based binding region (105) and/or a peptide based binding region (106);

c. Bringing the scaffold elements in solution, whereby the self-assembly of the scaffold elements (101) takes place, and

d. Obtaining the resulting three-dimensional scaffold.

In one embodiment the disclosure pertains to the three-dimensional scaffold produced by the method disclosed above.

The three-dimensional scaffold of the present disclosure is suitable for a large number of uses, such as the ones selected from the group consisting of use in biological imaging and labeling, use in templates for material synthesis, use in molecular sensing, use in diagnostic tools, use in molecular robotics and computing, use in synthetic biology, use in bottom-up nanofabrication, use in nanoscale devices, use in bioprocessing, and use in bioprinting, as well as combinations thereof.

Biological imaging and labeling involve techniques used to visualize and track biological molecules, cells, or tissues within living organisms or in vitro systems or biopsies of the aforementioned such as tissue sections, liquid biopsies, or lysates. These techniques may be used for understanding biological processes, diagnosing diseases, and developing treatments. Self-assembling scaffolds according to the disclosure may be used in enhancing biological imaging and labeling techniques. The self-assembling scaffolds of the present disclosure can spontaneously organize into ordered arrangements and can provide a framework for precise positioning and orientation of imaging or labeling agents, enhancing their efficiency and specificity in biological systems.

In the context of biological imaging and labeling, self-assembling scaffolds can serve several purposes:

- Spatial organization: The self-assembling scaffolds of the present disclosure can organize imaging or labeling agents into specific patterns or arrays, allowing precise spatial control over their distribution within biological samples. This spatial organization enables the visualization of molecular interactions, cellular structures, or tissue architecture with high resolution. Likewise, the self-assembling scaffolds of the present disclosure can organize target analytes into regular arrays, which may facilitate their detection. For example in the context of multi-plex (100s of analytes), high-plex (100s-1000s of analytes) or ultra-high plex (10000s of analytes) the self-assembling scaffolds of the present disclosure may be used to generate regular 3D arrays that position analytes (e.g. proteins or nucleic acids) in a flow cell taking advantage of the entire volume of said flow cell while ensuring ideal spacing of target analytes with respect to the resolving power of the readout. For example, such an array may be used for massively parallel sequencing of nucleic acid targets with a microscope-based readout and the targets may be positioned to have around 240-300nm distance in XY direction and about 700-1,000nm in Z direction. In another embodiment, the analytes may be proteins and the readout may be based on single molecule localization using for example DNA-PAINT or another blinking based technology (e.g. as disclosed in the European patent application with the application number 23192184.2 and/or in WO 2022/242887 A1, the complete contents of which are incorporated herein by reference) and the target-to-target distance may be in the range of few nanometers e.g. 5-10nm, 10-20nm, 10-100nm. In another embodiment, the self-assembling scaffold of the present disclosure further comprises a DNA-nanostructure-based NanoArray as described in WO 2022/207832 A1, the complete content of which is incorporated herein by reference, configured to capture analytes in certain capture bands. This embodiment enables capturing a large number of analytes as well as reading out a large number of analytes.

- Targeting and delivery: The self-assembling scaffolds of the present disclosure can be designed to target specific biological structures or cells. By incorporating targeting ligands or antibodies onto the scaffold surface, imaging or labeling agents can be delivered selectively to desired locations within the organism, enhancing imaging specificity and reducing off-target effects.

- Signal amplification: The self-assembling scaffolds of the present disclosure can be engineered to amplify imaging signals, thereby increasing the sensitivity of detection. For example, imaging agents attached to self-assembling nanoparticles can generate enhanced signals compared to freely diffusing agents, allowing for the detection of low-abundance biomolecules or cells.

- Biocompatibility and stability: The self-assembling scaffolds of the present disclosure can be designed to be biocompatible and stable in biological environments, minimizing cytotoxicity and degradation. This ensures the long-term viability of labeled cells or tissues for longitudinal imaging studies.

The self-assembling scaffolds of the present disclosure can be used in the process of creating new materials with desired properties through chemical, physical, or biological methods. The self-assembling scaffolds of the present disclosure can be utilized in material synthesis to engineer advanced materials with tailored structures and functions. Here's how self-assembling scaffolds enhance material synthesis:

- Controlled assembly: The self-assembling scaffolds of the present disclosure enable the precise control of material assembly at the nanoscale. By designing the molecular structure and interactions of scaffold components, researchers can dictate the arrangement of building blocks during the synthesis process. This control allows for the creation of materials with specific nanostructures, such as nanofibers, nanoparticles, or nanosheets, which are challenging to achieve using conventional methods.

- Hierarchical organization: The self-assembling scaffolds of the present disclosure facilitate hierarchical organization in material synthesis, where structures are built from multiple levels of organization. By tuning the properties of scaffold components, such as their size, shape, and surface chemistry, researchers can induce hierarchical self-assembly to generate complex materials with hierarchical structures. These materials often exhibit unique properties, such as enhanced mechanical strength, conductivity, or responsiveness to external stimuli.

- Functionalization: The self-assembling scaffolds of the present disclosure provide a versatile platform for functionalizing materials with diverse properties and functionalities. Functional molecules, such as polymers, nanoparticles, or biomolecules, can be incorporated into scaffold structures during synthesis, allowing for the introduction of specific properties, such as biocompatibility, catalytic activity, or optical responsiveness. This functionalization enhances the versatility and utility of synthesized materials for various applications, including biomedicine, electronics, and catalysis.

- Scalability and reproducibility: The self-assembling scaffolds of the present disclosure offer scalable and reproducible methods for material synthesis. Unlike traditional synthesis approaches that rely on complex reaction conditions or expensive equipment, self-assembly processes are often simple, cost-effective, and amenable to large-scale production. This scalability and reproducibility are essential for translating laboratory-developed materials into practical applications, such as industrial manufacturing or biomedical devices.

- Dynamic responsiveness: The self-assembling scaffolds of the present disclosure can exhibit dynamic responsiveness to external stimuli, allowing for reversible changes in material properties or structures. By incorporating stimuli-responsive components into scaffold designs, such as temperature-sensitive polymers or pH-responsive surfactants, researchers can engineer materials that undergo controlled structural transitions or phase changes in response to environmental cues. This dynamic behavior enables the development of smart materials with tunable properties for applications in sensing, drug delivery, and adaptive materials.

The self-assembling scaffolds also offer significant advantages in the field of molecular sensing and diagnostic tools, enhancing sensitivity, specificity, and versatility. Examples include:

- Enhanced signal amplification the self-assembling scaffolds of the present disclosure can facilitate signal amplification in molecular sensing assays. By incorporating multiple sensing elements or reporter molecules onto the scaffold surface, researchers can amplify the detection signal, enabling the sensitive detection of target molecules even at low concentrations. This enhanced sensitivity is crucial for diagnostic applications where early detection of biomarkers or pathogens is essential for disease diagnosis and monitoring.

- Multiplexed detection: The self-assembling scaffolds of the present disclosure enable multiplexed detection of multiple analytes in a single assay. By immobilizing different sensing elements or probes onto distinct regions of the scaffold, researchers can simultaneously detect multiple targets within a complex sample. This multiplexing capability enhances the throughput and efficiency of diagnostic assays, allowing for comprehensive analysis of biomolecular profiles or disease markers in biological samples.

- Spatial organization and immobilization: The self-assembling scaffolds of the present disclosure provide a platform for spatially organizing and immobilizing sensing elements with precise control. By designing the scaffold structure and surface chemistry, researchers can immobilize sensing probes in predefined locations or orientations, enhancing their accessibility to target molecules. This spatial organization improves the specificity and efficiency of molecular recognition, reducing nonspecific binding and background noise in diagnostic assays.

- Dynamic sensing platforms: The self-assembling scaffolds of the present disclosure can serve as dynamic sensing platforms that respond to changes in the surrounding environment or target analytes. By incorporating stimuli-responsive components into scaffold designs, such as molecular switches or responsive polymers, researchers can engineer sensors that undergo conformational changes or signal modulation in the presence of specific analytes. This dynamic responsiveness enables real-time monitoring of molecular interactions and facilitates the development of biosensors for dynamic physiological processes or point-of-care diagnostics.

- Biocompatibility and stability: The self-assembling scaffolds of the present disclosure are often biocompatible and stable in biological environments, making them suitable for in vivo sensing applications and diagnostic tools. These scaffolds can be designed to withstand physiological conditions and minimize nonspecific interactions with biological matrices, ensuring accurate and reliable detection of target molecules in complex biological samples. Additionally, their stability allows for long-term monitoring and continuous sensing in vivo, enabling applications such as implantable biosensors or wearable diagnostic devices.

The self-assembling scaffolds of the present disclosure may play a crucial role in advancing molecular robotics and computing by providing a programmable platform for the construction of dynamic molecular systems and devices. For example:

- Bottom-up assembly: The self-assembling scaffolds of the present disclosure enable the bottom-up assembly of complex molecular structures and devices from simple building blocks. By designing the molecular interactions and self-assembly pathways of scaffold components, researchers can orchestrate the precise assembly of molecular machines, robots, and circuits with defined structures and functions. This bottom-up approach allows for the creation of intricate molecular systems with nanoscale precision, overcoming the limitations of top-down fabrication techniques.

- Dynamic reconfigurability: The self-assembling scaffolds of the present disclosure offer dynamic reconfigurability, allowing for the reversible rearrangement of molecular components and structures in response to external stimuli or programming inputs. By incorporating stimuli-responsive elements into scaffold designs, such as switchable molecules or responsive polymers, researchers can engineer molecular systems that undergo programmable conformational changes or structural transformations on demand. This dynamic behavior enables the reprogramming and adaptation of molecular robots and computing devices for diverse applications and tasks.

- Information processing and computation: The self-assembling scaffolds of the present disclosure provide a platform for information processing and computation at the molecular scale. By designing scaffold architectures that support the organization and interaction of molecular components, researchers can implement computational algorithms and logic operations using molecular logic gates, circuits, and networks. These molecular computing devices can perform complex tasks such as data storage, pattern recognition, and decision-making, opening up new possibilities for molecular-level computing and information processing.

- Functional integration: The self-assembling scaffolds of the present disclosure facilitate the integration of functional components and modules into molecular robots and computing devices. By incorporating diverse functionalities, such as sensors, actuators, and communication modules, onto the scaffold surface or within scaffold structures, researchers can create multifunctional molecular systems capable of sensing, processing, and responding to environmental cues or external commands. This functional integration enhances the versatility and utility of molecular robots and computing devices for applications in nanomedicine, materials science, and beyond.

- Scalability and reproducibility: The self-assembling scaffolds of the present disclosure offer scalability and reproducibility in the fabrication of molecular robots and computing devices. Unlike conventional lithographic or micromanipulation techniques, which are limited by resolution and throughput, self-assembly processes are inherently parallel and scalable, allowing for the rapid and cost-effective production of large quantities of identical molecular structures. This scalability and reproducibility are essential for translating laboratory-developed prototypes into practical applications and commercial products.

Last, but not least, the self-assembling scaffolds of the present disclosure may also increasingly be utilized in the field of bioprinting, where they offer unique advantages for the fabrication of complex 3D biological structures and tissues. Examples include:

- Support and structure: The self-assembling scaffolds of the present disclosure provide a structural framework and support for bioprinted constructs. By organizing into ordered arrangements through self-assembly, these scaffolds offer a stable foundation for depositing bioinks and building complex 3D structures layer by layer. This support is crucial for maintaining the integrity and shape fidelity of bioprinted tissues and organs, preventing collapse or deformation during the printing process.

- Cell encapsulation and alignment: The self-assembling scaffolds of the present disclosure facilitate the encapsulation and alignment of cells within bioprinted constructs. By incorporating cell-adhesive molecules or peptides onto scaffold surfaces, researchers can promote cell adhesion and proliferation within the scaffold matrix, enhancing cell viability and functionality. Moreover, self-assembling scaffolds can align cells along specific orientations or patterns, mimicking the native architecture of tissues and facilitating cell-cell communication and organization.

- Biochemical cues and microenvironments: The self-assembling scaffolds of the present disclosure enable the incorporation of biochemical cues and microenvironments into bioprinted constructs. By functionalizing scaffold components with bioactive molecules, growth factors, or extracellular matrix proteins, researchers can create biomimetic environments that support cell growth, differentiation, and tissue regeneration. These bio-functionalized scaffolds enhance the biological performance and functionality of bioprinted tissues, promoting tissue maturation and integration within the host environment.

- Dynamic remodeling and degradation: The self-assembling scaffolds of the present disclosure can exhibit dynamic remodeling and degradation properties, allowing for the gradual remodeling and integration of bioprinted tissues into host tissues. By incorporating degradable components or responsive polymers into scaffold designs, researchers can engineer scaffolds that undergo controlled degradation and remodeling in response to physiological cues or cellular activity. This dynamic behavior enables the gradual replacement of scaffold materials with native extracellular matrix, promoting tissue maturation and vascularization in bioprinted constructs.

- Multimaterial printing: The self-assembling scaffolds of the present disclosure enable multimaterial bioprinting, where multiple bioinks or scaffold materials are deposited simultaneously to create complex heterogeneous tissues and organs. By designing scaffold structures that accommodate the deposition of multiple materials, researchers can create bioprinted constructs with spatially defined regions or gradients of biochemical and mechanical properties. This multimaterial printing capability enables the fabrication of functional tissues with heterogeneous cell populations, vascular networks, and specialized microenvironments, advancing the development of complex organ models and regenerative therapies.

In yet another embodiment the three-dimensional scaffold of the present disclosure for use as a medicament are encompassed, such as use in tissue and/or cellular repair, tissue and/or cellular engineering, drug delivery, wound treatment, bone reconstruction, building artificial organs, as well as combinations thereof.

Examples include:

The three-dimensional scaffold of the present disclosure is suitable for use to repair damaged cardiac tissue after a heart attack. The scaffold, composed of peptide sequences that promote cell adhesion and tissue regeneration, is injected into the infarcted area of the heart. Once injected, the scaffold self-assembles into a 3D matrix that provides mechanical support and creates a conducive microenvironment for the recruitment and proliferation of cardiac cells. Over time, the scaffold degrades, allowing the newly formed tissue to integrate with the surrounding myocardium, ultimately restoring heart function.

The three-dimensional scaffold of the present disclosure can be employed in neural tissue engineering to promote axonal growth and regeneration in spinal cord injuries. The scaffold mimics the architecture of the extracellular matrix and provides physical guidance cues for regenerating axons. Neural cells seeded onto the scaffold adhere and extend their neurites along the aligned nanofibers, facilitating axonal regeneration and functional recovery in injured spinal cord tissue.

The three-dimensional scaffold of the present disclosure can be utilized for targeted drug delivery to treat cancer. The scaffold can encapsulate hydrophobic chemotherapeutic drugs within their core. Functional groups on the micelle surface can be modified with targeting ligands that specifically recognize cancer cells. Upon administration, the scaffold accumulates at the tumor site via passive or active targeting mechanisms and release the encapsulated drugs, resulting in localized and efficient chemotherapy with reduced systemic toxicity.

The three-dimensional scaffold of the present disclosure can be applied as advanced wound dressings for diabetic foot ulcers. The scaffold, containing bioactive peptide sequences that promote cell proliferation and angiogenesis, is applied topically to the wound bed. The scaffold self-assembles upon contact with wound exudate, forming a protective barrier that maintains a moist wound environment and accelerates tissue regeneration. The bioactive peptides in the scaffold stimulate the migration and proliferation of keratinocytes, fibroblasts, and endothelial cells, promoting wound closure and vascularization. The scaffold may in this case further be doted with antibiotics to inhibit bacterial growth and/or comprise Fibrinogen to facilitate integration of the scaffold with the natural clotting process.

The three-dimensional scaffold of the present disclosure can be used for bone tissue engineering and reconstruction. The scaffolds, comprising biocompatible ceramic nanoparticles that self-assemble into porous structures, provide a biomimetic environment for osteogenic cell seeding and bone formation. Osteogenic cells, such as mesenchymal stem cells, adhere to the scaffold surface and differentiate into osteoblasts, depositing new bone matrix within the scaffold pores. Over time, the scaffold gradually degrades, leaving behind newly formed bone tissue that integrates with the surrounding bone, facilitating bone regeneration and repair.

The three-dimensional scaffold of the present disclosure can be engineered as functional units for building artificial organs or parts thereof, such as kidneys. Kidney organoids, derived from induced pluripotent stem cells or primary renal cells, are cultured within self-assembling scaffolds that mimic the extracellular matrix composition of native kidney tissue. The scaffold provides structural support and biochemical cues (e.g. the scaffold is decorated with suitable growth factors, hormones, serum factors for example) for organoid development and maturation. As the organoids grow and differentiate, they recapitulate key features of the kidney nephron, including filtration, reabsorption, and secretion functions, ultimately forming functional units that can be assembled into a bioengineered kidney for transplantation or in vitro drug testing.

In yet another embodiment the disclosure pertains to a kit comprising the three-dimensional scaffold as disclosed hereinunder further comprising a buffer, a package leaflet, an applicator, an administration device, a mixing device, a manual, a device for induction of polymerization, a dye, and a hydrogel-matrix, as well as combinations thereof.

Sequences of the disclosure

| SEQ ID | Name | Isoform (Uni-Prot-Identifier) | Length [aa] | MW [Da] |
|---|---|---|---|---|
| SEQ ID NO: 1 | Titin 01 | Q8WZ42-1 | 34,350 | 3,816,030 |
| SEQ ID NO: 2 | Titin 02 | Q8WZ42-2 | 34,258 | 3,805,708 |

(continued)

| SEQ ID | Name | Isoform (Uni-Prot-Identifier) | Length [aa] | MW [Da] |
|---|---|---|---|---|
| SEQ ID NO: 3 | Titin 03 | Q8WZ42-3 | 26,926 | 2,992,939 |
| SEQ ID NO: 4 | Titin 04 | Q8WZ42-4 | 33,445 | 3,716,027 |
| SEQ ID NO: 5 | Titin 05 | Q8WZ42-5 | 32,900 | 3,653,085 |
| SEQ ID NO: 6 | Titin 06 | Q8WZ42-6 | 5,604 | 631,567 |
| SEQ ID NO: 7 | Titin 07 | Q8WZ42-7 | 33,615 | 3,734,648 |
| SEQ ID NO: 8 | Titin 08 | Q8WZ42-8 | 34,475 | 3,829,846 |
| SEQ ID NO: 9 | Titin 09 | Q8WZ42-9 | 27,118 | 3,013,957 |
| SEQ ID NO: 10 | Titin 10 | Q8WZ42-10 | 27,051 | 3,006,755 |
| SEQ ID NO: 11 | Titin 11 | Q8WZ42-11 | 33,423 | 3,713,600 |
| SEQ ID NO: 12 | Titin 12 | Q8WZ42-12 | 35,991 | 3,994,625 |
| SEQ ID NO: 13 | Titin 13 | Q8WZ42-13 | 34,484 | 3,821,069 |
| SEQ ID NO: 14 | Nebulin | Q05C45 | 287 | 33,228 |
| SEQ ID NO: 15 | Obscurin 01 | Q5VST9-1 | 7,968 | 868,484 |
| SEQ ID NO: 16 | Obscurin 02 | Q5VST9-2 | 7,969 | 868,571 |
| SEQ ID NO: 17 | Obscurin 03 | Q5VST9-3 | 6,620 | 721,547 |
| SEQ ID NO: 18 | Obscurin 05 | Q5VST9-5 | 3,911 | 427,046 |
| SEQ ID NO: 19 | Obscurin 06 | Q5VST9-6 | 8,483 | 924,971 |
| SEQ ID NO: 20 | Dystrophin 01 | P11532-1 | 3,685 | 426,750 |
| SEQ ID NO: 21 | Dystrophin 02 | P11532-2 | 2,344 | 271,391 |
| SEQ ID NO: 22 | Dystrophin 03 | P11532-3 | 2,341 | 271,040 |
| SEQ ID NO: 23 | Dystrophin 04 | P11532-4 | 3,677 | 425,640 |
| SEQ ID NO: 24 | Dystrophin 05 | P11532-5 | 622 | 70,750 |

(continued)

| SEQ ID | Name | Isoform (Uni-Prot-Identifier) | Length [aa] | MW [Da] |
|---|---|---|---|---|
| SEQ ID NO: 25 | Dystrophin 06 | P11532-6 | 635 | 72,191 |
| SEQ ID NO: 26 | Dystrophin 07 | P11532-7 | 617 | 70,375 |
| SEQ ID NO: 27 | Dystrophin 08 | P11532-8 | 604 | 68,934 |
| SEQ ID NO: 28 | Dystrophin 09 | P11532-9 | 525 | 59,769 |
| SEQ ID NO: 29 | Dystrophin 11 | P11532-11 | 3,681 | 426,001 |
| SEQ ID NO: 30 | Dystrophin 12 | P11532-12 | 1,225 | 141,371 |
| SEQ ID NO: 31 | Dystrophin 13 | P11532-13 | 1,115 | 128,950 |
| SEQ ID NO: 32 | Dystrophin 14 | P11532-14 | 1,243 | 143,187 |
| SEQ ID NO: 33 | Dystrophin 15 | P11532-15 | 1,230 | 141,746 |
| SEQ ID NO: 34 | Dystrophin 16 | P11532-16 | 1,133 | 130,765 |
| SEQ ID NO: 35 | Dystrophin 17 | P11532-17 | 956 | 109,942 |
| SEQ ID NO: 36 | Dystrophin 18 | P11532-18 | 340 | 39,254 |
| SEQ ID NO: 37 | Sequence A: Derived from Human sickle cell beta-globin mRNA (European Nucleotide Archive, Seq No. M25079.1) | | | |
| SEQ ID NO: 38 | Sequence A': Complementary sequence to A. | | | |

as well as isoforms, variants, and fragments thereof.

**Definitions**

Titin (SEQ ID NO: 1-13), also known as connectin, is a giant protein found in vertebrate muscle cells. It is the largest known protein, with a molecular weight ranging from 3 to 4 megadaltons (MDa) and a length of up to 1 micron. Titin spans half the length of a sarcomere, which is the basic contractile unit of muscle, extending from the Z-disc to the M-line.

Functionally, titin serves several critical roles in muscle physiology:

- Elasticity: Titin acts as a molecular spring, providing passive elasticity to muscle fibers and contributing to their ability to stretch and recoil during contraction and relaxation. Its extensible structure allows it to withstand and transmit forces generated during muscle contraction.

- Sarcomere Assembly and Stability: Titin anchors myosin thick filaments to the Z-disc and helps maintain the structural integrity of sarcomeres. It interacts with other structural proteins, such as actin and myosin, to stabilize the sarcomeric structure and ensure proper alignment of contractile filaments.

- Regulation of Muscle Function: Titin plays a role in regulating muscle function by modulating the passive tension of muscle fibers in response to changes in muscle length and stretching. It contributes to the passive force developed during muscle lengthening and helps optimize muscle performance across a range of physiological conditions.

- Signaling: Titin has been implicated in signaling pathways involved in muscle growth, development, and adaptation to mechanical stress. It interacts with various proteins and signaling molecules, influencing processes such as muscle hypertrophy, mechanotrans-duction, and gene expression.

"PEVK" is an acronym representing a region found in the giant elastic protein titin, which is present in muscle tissues. The PEVK region stands for Proline (P), Glutamate (E), Valine (V), and Lysine (K). These amino acids are part of the repetitive sequence found in the I-band region of titin, which contributes to the extensibility and elasticity of the protein. The PEVK region is known for its high content of proline and glutamate residues, which are thought to impart flexibility to the titin molecule, allowing it to stretch and recoil during muscle contraction and relaxation.

Nebulin (SEQ ID NO: 14), is a large protein found in skeletal muscle fibers, similar to titin. It plays a crucial role in regulating the length of the thin filaments in muscle sarcomeres, thereby contributing to muscle contraction.

Obscurin (SEQ ID NO: 15-19), is a giant sarcomeric protein found in skeletal and cardiac muscle. It interacts with various structural and signaling proteins, including titin, and is involved in maintaining the structural integrity of muscle cells and regulating contractile function.

Dystrophin (SEQ ID NO: 20-36), is a cytoskeletal protein found primarily in muscle cells. It plays a crucial role in linking the cytoskeleton to the extracellular matrix and stabilizing the sarco-lemma (cell membrane) during muscle contraction. Mutations in the dystrophin gene are associated with Duchenne and Becker muscular dystrophy.

The terms "protein isomer" and "protein variant" as used hereinunder refer to different concepts related to proteins, particularly regarding variations in their structure, sequence, or function. Here's a brief explanation of each: A "protein isomer" or "isomer" refers to different spatial arrangements of the atoms within a protein molecule while retaining the same chemical composition. Protein isomers can result from various types of isomerization processes, such as conformational changes, cis-trans isomerization of peptide bonds, or changes in stereochemistry of amino acid residues. For example, in the case of conformational isomers, proteins may exist in multiple conformations or structural states due to flexibility or dynamic motion of their constituent amino acid residues. These conformational isomers can have distinct shapes or folding patterns while having identical amino acid sequences. Protein isomers may also include stereoisomers, which have the same sequence of amino acids but differ in the spatial arrangement of their atoms. An example of this is the cis-trans isomerization of proline residues within a protein chain, which can lead to different spatial arrangements of the polypeptide backbone.

A "protein variant" refers hereinunder to different forms or versions of a protein that arise due to genetic variations, post-translational modifications, alternative splicing, or other processes that result in changes to the amino acid sequence or structure of the protein. Protein variants can have alterations in their amino acid sequence, such as single nucleotide polymorphisms (SNPs) leading to amino acid substitutions, insertions, deletions, or truncations. These variations can affect the protein's structure, function, stability, or interactions with other molecules. In one embodiment a protein is considered a variant if it has a sequence identity with any of the sequences disclosed hereinunder of at least 50%, at least 55%, at least 60%, at least 65%, at least 70%, at least 75%, at least 80%, at least 85%, at least 90%, at least 95%, at least 96%, at least 97%, at least 98%, at least 99.0%, at least 99.3%, at least 99.5%, at least 99,9%, or at least 99,99%. Additionally, protein variants can arise from post-translational modifications (PTMs) such as phosphorylation, glycosylation, acetylation, or proteolytic cleavage, which can modify specific amino acid residues or alter the protein's chemical properties and biological activity.

The term "essentially complementary nucleic acid sequences" refers to two strands of nucleic acids (DNA or RNA) that possess sequences that can base pair with each other according to the rules of Watson-Crick base pairing. In other words, the sequences are complementary in such a way that adenine (A) pairs with thymine (T) in DNA, adenine (A) pairs with uracil (U) in RNA, and guanine (G) pairs with cytosine (C). The term "essentially complementary" implies that the sequences may not be perfectly complementary throughout their entire length but share enough complementary bases to form stable duplex structures. This could include sequences with occasional mismatches, bulges, loops, or non-Watson-Crick base pairs, as long as the overall complementarity allows for stable hybridization and formation of a double-stranded structure under the selected hybridization conditions. Essentially complementary nucleic acid sequences are often used in various molecular biology techniques such as PCR (polymerase chain reaction), DNA sequencing, hybridization assays, and molecular cloning, where specific sequences need to anneal or hybridize with each other for the desired outcome. In one embodiment the "essentially complementary nucleic acid sequences" comprise at least 80%, at least 85%, at least 90%, at least 95%, or at least 99% of complementary nucleobases.

"Specific hybridization" of nucleic acids refers to the selective binding of two nucleic acid strands with complementary sequences to form a stable duplex structure. In specific hybridization, the base pairing between nucleotides follows the rules of Watson-Crick base pairing, where adenine (A) pairs with thymine (T) in DNA or uracil (U) in RNA, and guanine (G) pairs with cytosine (C).

The term "specific hybridization" indicates that the hybridization occurs only between nucleic acid strands with sequences that are sufficiently complementary to each other, allowing for precise and predictable interactions. Specific hybridization relies on the complementarity of nucleic acid sequences, where each base on one strand pairs specifically with its complementary base on the other strand. Specific hybridization allows under the selected hybridization conditions the demarcation of one hybridization pair (e.g. A with A') in a solution from another pair (e.g. B with B') and does not apply to "unspecific hybridization" (e.g. A with B).

In the context of the present disclosure, a "barcode" refers to a short, unique sequence of nucleotides (DNA or RNA) that serves as a molecular identifier or tag. These barcodes are incorporated into nucleic acid molecules to distinguish and identify them in high-throughput experiments, such as DNA sequencing, gene expression analysis, or multiplexed assays. The barcode sequence is typically designed to be short enough to be easily read and recognized, but long enough to provide sufficient diversity for distinguishing different samples or molecules. Barcodes can be added to nucleic acids during library preparation or synthesis using various molecular biology techniques, such as PCR (polymerase chain reaction), ligation, or synthesis with modified nucleotides.

In the context of this disclosure "programmable" refers to the ability to design and manipulate nucleic acid sequences in a precise and predictable manner to perform specific tasks or functions. DNA molecules are programmable because the sequence of nucleotides in a DNA strand can be precisely engineered to encode information or instructions for carrying out desired molecular processes. By designing complementary sequences and exploiting the principles of Watson-Crick base pairing, researchers can create nucleic acid structures with predetermined shapes, properties, and functionalities. In nucleic acid nanotechnology, programmable DNA sequences are used to construct nanostructures and nanodevices through self-assembly or molecular recognition processes. These nanostructures can be designed to have specific shapes, sizes, and surface properties, making them useful for applications such as drug delivery, biosensing, and molecular imaging. In DNA computing, programmable DNA sequences serve as the information carriers and processing units for performing computational tasks. By encoding logic gates, algorithms, or computational instructions into DNA sequences, researchers can use DNA molecules to perform parallel computations and solve complex computational problems.

In the context of this disclosure, "PDI" stands for "Polydispersity Index." The polydispersity index is a measure of the distribution of molecular weights in a polymer sample. It provides information about the spread or variability of molecular weights within the sample. The polydispersity index is calculated as the weight-average molecular weight (Mw) divided by the number-average molecular weight (Mn). Mathematically, it can be expressed as:

$$\mathrm{PDI} = \frac{M_w}{M_n}$$

wherein:

$M_w$ is the weight-average molecular weight, calculated by summing the products of the molecular weight of each polymer chain and its respective weight fraction in the sample; and

$M_n$ is the number-average molecular weight, calculated by summing the products of the molecular weight of each polymer chain and its respective number fraction in the sample.

A polydispersity index of 1 indicates a monodisperse polymer sample, where all polymer chains have the same molecular weight. A higher polydispersity index indicates a broader distribution of molecular weights, with greater variability among the polymer chains in the sample.

Polydispersity index is an important parameter in polymer characterization, as it affects the physical properties and performance of polymer materials. Polymers with lower polydispersity indices often exhibit more uniform properties and behavior, while polymers with higher polydispersity indices may have more variable properties and performance due to the presence of a wider range of molecular weights.

In one embodiment the PDI of the three-dimensional scaffold may be at least 1.0, at least 1.1, at least 1.2, at least 1.5, at least 2.0, at least 3.0, at least 4.0, at least 5.0, at least 6.0, at least 7.0, at least 8.0, at least 9.0, or at least 10.0.

To test the "tensile strength" of a protein, a technique called "mechanical stretching" or "tensile testing" may be employed. This method involves applying controlled mechanical force to a protein sample and measuring its response to deformation. While this method is often used for testing the mechanical properties of materials such as polymers and metals, it can also be adapted for proteins.

The main steps to measure the tensile strength of a protein are:

- Sample Preparation: The protein sample needs to be prepared in a form suitable for tensile testing. This may involve isolating the protein of interest and forming it into a consistent and homogeneous structure, such as fibers, films, or hydrogels. The sample should be free from defects or impurities that could affect the test results.

- Mounting the Sample: The prepared protein sample is mounted onto a testing apparatus equipped with grips or clamps that can securely hold the sample. Care should be taken to ensure that the sample is aligned properly and that the grips apply an even distribution of force to prevent premature failure or deformation.

- Application of Force: A controlled mechanical force is applied to the protein sample using the testing apparatus. This force is gradually increased at a constant rate while monitoring the deformation of the sample. The applied force can be measured directly using load cells integrated into the testing machine.

- Measurement of Deformation: During the tensile test, the deformation of the protein sample is typically measured using displacement sensors or extensometers. These devices track changes in the sample's length or dimensions as it undergoes stretching or elongation.

- Analysis of Stress-Strain Curve: The data obtained from the tensile test, including applied force and deformation measurements, are used to generate a stress-strain curve for the protein sample. The stress is calculated as the force applied per unit area, while the strain is the ratio of the change in length to the original length of the sample. The tensile strength of the protein, which represents its resistance to deformation under tension, can be determined from the maximum stress observed on the stress-strain curve.

Measuring the extensibility of a protein involves quantifying its ability to undergo deformation or elongation in response to an applied force. Extensibility is often assessed experimentally using techniques such as atomic force microscopy (AFM), optical tweezers, or mechanical stretching assays.

AFM is a powerful technique that allows for the direct manipulation and measurement of the mechanical properties of individual protein molecules. In AFM-based stretching experiments, a protein molecule of interest is typically immobilized on a surface, and a cantilever with a sharp tip is brought into contact with the protein. As the cantilever is moved, it pulls or stretches the protein molecule, and the resulting force-displacement curve is recorded. From this curve, parameters such as the protein's extensibility, stiffness, and unfolding forces can be determined.

Measuring the toughness of a protein involves assessing its ability to absorb energy and deform without fracturing when subjected to mechanical stress. Toughness is a key mechanical property that reflects the resistance of a protein to fracture or failure under load. AFM-based techniques can be used to measure the toughness of proteins by subjecting individual protein molecules to mechanical deformation and monitoring their response. In AFM force spectroscopy experiments, a protein molecule is immobilized on a surface, and a cantilever with a sharp tip is brought into contact with the protein. The cantilever is then moved to apply a controlled force to the protein, causing it to deform. The toughness of the protein can be determined from the force-displacement curve obtained during the stretching or unfolding process. A protein with higher toughness will require more energy to deform and will exhibit a higher resistance to fracture.

Further methods for testing the physical properties such as tensile strength, extensibility and toughness of a protein can be found in Bowen et al., "Microbial production of megadalton titin yields fibers with advantageous mechanical properties", Nature communications, https://doi.org/10.1038/s41467-021-25360-6.

Examples

**Example 1 - Production of a scaffold element (101)**

**1.1 Strains and growth conditions.**

E. coli NEB 10-beta (NEB10β) is used for all plasmid cloning and protein production. For all cloning, E. coli strains were cultured in Terrific Broth (TB) containing 24 g/L yeast extract, 20 g/L tryptone, 0.4% v/v glycerol, 17 mM $KH_2PO_4$, and 72 mM $K_2HPO_4$ at 37 °C with appropriate antibiotics (50 μg/mL kanamycin). M9 glucose medium with tryptone supplement (2% w/v glucose, 1× M9 Salts, 75 mM MOPS pH 7.4, 12 g/L tryptone, 5 mM sodium citrate, 2 mM $MgSO_4$ $7H_2O$, 100 μM $FeSO_4$ $7H_2O$, 100 μM $CaCl_2$ $2H_2O$, 3 μM thiamine, 1× micronutrients [40 μM $ZnSO_4$ $7H2O$, 20 μM $CuSO_4$ $5H_2O$, 10 μM $MnCl_2$ $4H_2O$, 4 μM $H_3BO_3$, 0.4 μM $(NH_4)6Mo_7O_{24}$ $4H_2O$, and 0.3 μM $CoCl_2$ $6H_2O$]) is used for protein production in bioreactors.

**1.2 Chemicals and reagents.**

Unless otherwise noted, all chemicals and reagents are obtained from MilliporeSigma. Plasmid purification and gel extraction kits are purchased from iNtRON Biotechnology. FastDigest restriction enzymes and T4 DNA ligase are purchased from Thermo Fisher Scientific and used for all digestions and ligations following manufacturer protocols.

**1.3 Construction and expression optimization of Titin-based peptide components (102) for scaffold element (101).**

Coding sequence of titin SEQ ID NO: 1 is inserted between KpnI and Kpn2I restriction sites of modified BglBricks63 vectors containing gp41-1C and gp41-1N SIs under the control of a PBAD promoter or a PLacO1 promoter, yielding expression plasmids.

**1.4 Bioproduction in shake flask cultures.**

Overnight seed cultures of 50 mL TB medium are inoculated with single colonies carrying the desired construct. These seed cultures are then used to inoculate cultures of 500 mL TB in 2 L Erlenmeyer flasks at an initial OD600 of 0.08. Cultures are placed on reciprocal shakers at 350 rpm at 37 °C until OD600 reached 3.0, at which point the corresponding inducer are added. Cultures are then continued at 37 °C for 20 h.

**1.5 Bioproduction in fed-batch bioreactors.**

Titin-based peptide components (102) are ultimately produced in 2 L fed-batch bioreactors (Bioflo120, Eppendorf). Transformants containing the plasmid are cultured overnight in 50 mL TB medium at 37 °C on an orbital shaker. The overnight cultures are then used to inoculate an autoclaved 2 L Bioflo120 heat-blanketed bioreactor containing 1.5 L M9 glucose medium with tryptone supplement (see above). Antifoam 204 is added as needed to minimize foaming (approximately 0.01% v/v). Agitation and airflow are regulated to maintain approximately 70% dissolved oxygen (DO).
After consumption of the initial 0.5% w/v glucose (as judged by $\Delta$DO), a sterile substrate feed (20% w/v glucose, 48 g/L tryptone, and 10 g/L $MgSO_4$ $7H_2O$) is initiated to maintain a linear growth rate. Reactors are induced at OD600 =70 by addition of 1 mM IPTG, and the incubation temperature is reduced to 30 °C.
Cultures are collected six hours after induction.

**1.6 Protein purification.**

Titin-based peptide components (102) are purified by dissolving cell pellets in an aqueous lysis buffer (50 mM Tris, 50 mM NaCl, 1 mM PMSF, and 300 $\mu$g/mL lysozyme). After stirring for 30 min at 4 °C, 5 mM MgCl2 and 5 $\mu$g/mL DNasel are added, and the mixture was sonicated with stirring on ice for 10 min (5s on, 10s off). After sonication, NaCl and imidazole were added to final concentrations of 300 mM and 10 mM, respectively. The mixture is centrifuged at 25,000 $\times$ g for 30 min at 4 °C, followed by 75,000 $\times$ g for 30 min at 4 °C. Cleared supernatant is then filtered and applied to a series of six HisTrap HP 5 mL columns at 2 mL/min. Loaded columns are washed with 2 column volumes of wash buffer (50 mM Tris,300 mM NaCl, 10 mM imidazole), then washed with 2 column volumes wash buffer with 50 mM imidazole, and finally eluted with wash buffer with 300 mM imidazole.
After purification by affinity chromatography, the proteins (i.e. the titin-based peptide components (102)) were fully dialyzed to 5 mM ammonium bicarbonate at 4 °C using 10 kDa MWCO snakeskin dialysis tubing (Thermo Fisher Scientific).

**1.7. Addition of two nucleic acid components (103,104).**

Two nucleic acid components of SEQ ID NO: 37 and 38 are added to either end of the titin-based peptide components (102) via strain-promoted alkyne-azide cycloaddition (SPAAC).
The final scaffold element (101) is schematically depicted in figure 11.

**Example 2** - **Formation of the three-dimensional scaffold.**

About 0.5% (w/v) of scaffold elements (101) are added to a sodium-phosphate-buffer containing water (pH 7.5) at 20°C. The solution is moderately stirred and then stored for 6 hours for self-assembly until the three-dimensional scaffold has been formed.
The three-dimensional scaffold may be shaped into forms by using the appropriate containers for self-assembly. Or by cutting the three-dimensional scaffold after formation, for example with a laser-cutter.

**Claims**

1. A three-dimensional scaffold (100) comprising,

a plurality of scaffold elements (101), each scaffold element comprising at least one peptide component (102) consisting of a stretch of amino acids and at least two nucleic acid components (103,104), wherein the at least two nucleic acid components (103,104) of the plurality of scaffold elements (101) are configured to mediate the self-assembly of the scaffold (100).

2. The three-dimensional scaffold according to claim 1, wherein the at least two nucleic acid components (103,104) comprise a first nucleic acid component and second nucleic acid component, the second nucleic acid component being different to the first nucleic acid component, wherein the first and second nucleic acid component are preferably configured to hybridize to different targets.

3. The three-dimensional scaffold according to any of the previous claims, wherein the at least one peptide component (102) does not comprise PNA.

4. The three-dimensional scaffold according to any of the previous claims, wherein the at least one peptide component (102) consists of a stretch of amino acids connected by peptide-bonds.

5. The three-dimensional scaffold according to any of the previous claims, wherein at least two nucleic acid components (103,104) are positioned at predetermined positions of the scaffold element (101).

6. The three-dimensional scaffold according to any of the previous claims, wherein each of the at least two nucleic acid components (103,104) comprise from 9 to 25 nucleobases.

7. The three-dimensional scaffold according to any of the previous claims, wherein each of the at least two nucleic acid components (103,104) are encoded to specifically hybridize with at least one of the nucleic acid components of another scaffold element (101a, 101b), thereby mediating the self-assembly of the scaffold (100).

8. The three-dimensional scaffold according to any of the previous claims, wherein the at least one peptide component (102) is characterized at a molecular weight (MW) of 2.5 MDa by at least one of the following features:

   • a longitudinal tensile strength of at least 350 MPa,
   • a tensile modulus of at least 3.5 GPa,
   • an extensibility of at least 45%,
   • a toughness of at least 120 Mj/m$^3$,
   • having a length of at least 0.5 $\mu$m,
   • comprising at least 3,000 amino acids.

9. The three-dimensional scaffold according to any of the previous claims, wherein the at least one peptide component (102) comprises at least 100 Ig-like domains and/or at least 10 proline-glutamate-valine-lysine (PEVK)-motifs.

10. The three-dimensional scaffold according to any of the previous claims, wherein the at least one peptide component (102) comprises a peptide selected from the group comprising SEQ ID NO: 1 to 36, as well as variants thereof with a sequence identity of at least 90%, and isoforms thereof.

11. The three-dimensional scaffold according to any of the previous claims, further comprising at least one nucleic acid based binding region (105) and/or at least one peptide-based binding region (106).

12. A method for producing a three-dimensional scaffold comprising the steps of

   a. Providing a plurality of scaffold elements (101) comprising at least one peptide component (102) and at least two nucleic acid components (103,104),
   b. Wherein, optionally, at least one of the scaffold elements (101) further comprises a binding element selected from a nucleic acid based binding region (105) and/or a peptide based binding region (106),
   c. Bringing the scaffold elements in solution, whereby the self-assembly of the scaffold elements (101) takes place, and

d. Obtaining a three-dimensional scaffold.

13. Use of the three-dimensional scaffold according to any of claims 1 to 11, selected from the group consisting of use in biological imaging and labeling, use in templates for material synthesis, use in molecular sensing, use in diagnostic tools, use in molecular robotics and computing, use in synthetic biology, use in bottom-up nanofabrication, use in nanoscale devices, use in bioprocessing, and use in bioprinting, as well as combinations thereof.

14. The three-dimensional scaffold according to any of claims 1 to 11 for use as a medicament, such as for use in the treatment of tissue and/or cellular repair, tissue and/or cellular engineering, drug delivery, wound, bone reconstruction, building artificial organs, as well as combinations thereof.

15. A kit comprising the three-dimensional scaffold according to any of claims 1 to 11 further comprising at least one item selected from the group consisting of a buffer, a package leaflet, an applicator, an administration device, a mixing device, a manual, a device for induction of polymerization, a dye, and a hydrogel-matrix, as well as combinations thereof.

100

101

103

102

104

101

Figure 1

**FIGURE 2**

**FIGURE 3**

**FIGURE 4**

# FIGURE 5

103

105a

108

105b

109

104

**FIGURE 6**

**FIGURE 7**

100a

100b

100c

100d

100e

100f

**FIGURE 8**

**FIGURE 9**

**FIGURE 10**

SEQ ID NO: 37 -CCCCC- SEQ ID NO: 37    SEQ ID NO: 38 -CCCCC- SEQ ID NO: 38

SEQ ID NO: 1

103 — 104

102 —

101

FIGURE 11

FIGURE 12

| | Europäisches Patentamt European Patent Office Office européen des brevets | **EUROPEAN SEARCH REPORT** | **Application Number** EP 24 16 9066 |
|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Macculloch Tara ET AL: "Emerging applications of peptide-oligonucleotide conjugates: bioactive scaffolds, self-assembling systems, and hybrid nanomaterials", Organic & biomolecular chemistry, 13 February 2019 (2019-02-13), pages 1668-1682, XP055896207, England DOI: 10.1039/c8ob02436g Retrieved from the Internet: URL:https://pubs.rsc.org/en/content/getaut horversionpdf/C8OB02436G [retrieved on 2022-02-28] * abstract * * page 3958, left-hand column, paragraph 1 - right-hand column, paragraph 1 * * page 3960, last paragraph * ----- | 1-8, 11-15 | INV. A61L27/22 A61K31/00 A61L27/54 C12N5/00 |
| A | CHUANG LI ET AL: "Rapid Formation of a Supramolecular Polypeptide-DNA Hydrogel for In?Situ Three-Dimensional Multilayer Bioprinting", ANGEWANDTE CHEMIE INTERNATIONAL EDITION, vol. 54, no. 13, 5 February 2015 (2015-02-05), pages 3957-3961, XP055489253, Hoboken, USA ISSN: 1433-7851, DOI: 10.1002/anie.201411383 * abstract * * page 6, paragraph 2 * ----- -/-- | 1-15 | TECHNICAL FIELDS SEARCHED (IPC) A61L C12N A61K |

The present search report has been drawn up for all claims

| Place of search | Date of completion of the search | Examiner |
|---|---|---|
| The Hague | 19 September 2024 | Dudás, Eszter |

EPO FORM 1503 03.82 (P04C01)

Europäisches Patentamt
European Patent Office
Office européen des brevets

# EUROPEAN SEARCH REPORT

**Application Number**

EP 24 16 9066

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | DHRUV MENON ET AL: "Designer, Programmable DNA-peptide hybrid materials with emergent properties to probe and modulate biological systems", CHEMBIOCHEM, JOHN WILEY & SONS, INC, HOBOKEN, USA, vol. 24, no. 5, 18 January 2023 (2023-01-18), page n/a, XP072598025, ISSN: 1439-4227, DOI: 10.1002/CBIC.202200580 * abstract * * page 7 - page 12 * ----- | 1-15 | |

TECHNICAL FIELDS
SEARCHED (IPC)

The present search report has been drawn up for all claims

| Place of search | Date of completion of the search | Examiner |
|---|---|---|
| The Hague | 19 September 2024 | Dudás, Eszter |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

........................................................................
& : member of the same patent family, corresponding document

EPO FORM 1503 03.82 (P04C01)

**REFERENCES CITED IN THE DESCRIPTION**

**Patent documents cited in the description**

- EP 23192184
- WO 2022242887 A1
- WO 2022207832 A1